Europäisches Patentamt

European Patent Office

Office européen des brevets

(11) Veröffentlichungsnummer: **0 317 854 B1**

## (12) EUROPÄISCHE PATENTSCHRIFT

(45) Veröffentlichungstag der Patentschrift: **20.10.93**

(21) Anmeldenummer: **88118888.2**

(22) Anmeldetag: **12.11.88**

(51) Int. Cl.⁵: **C12M 1/00**, C12M 1/08

(54) **Verfahren zur abgasfreien Begasung von Fermentationsmedien.**

(30) Priorität: **23.11.87 DE 3739621**

(43) Veröffentlichungstag der Anmeldung:
**31.05.89 Patentblatt 89/22**

(45) Bekanntmachung des Hinweises auf die
Patenterteilung:
**20.10.93 Patentblatt 93/42**

(84) Benannte Vertragsstaaten:
**AT BE CH DE FR GB IT LI NL**

(56) Entgegenhaltungen:
**EP-A- 0 041 702**
**EP-A- 0 164 813**
**FR-A- 2 588 271**
**US-A- 1 732 921**
**US-A- 3 846 245**

(73) Patentinhaber: **HOECHST AKTIENGESELL-
SCHAFT**

**D-65926 Frankfurt(DE)**

(72) Erfinder: **Buchholz, Rainer, Dr.**
**Grosser Ring 38**
**D-5239 Unnau(DE)**
Erfinder: **Schäfer, Hans**
**Am Rehsteig 15**
**D-6232 Bad Soden am Taunus(DE)**
Erfinder: **Schlingmann, Merten, Prof. Dr.**
**Schneihainer Strasse 32a**
**D-6240 Königstein/Taunus(DE)**
Erfinder: **Siegemund, Günter, Dr.**
**Frankfurter Strasse 21**
**D-6238 Hofheim am Taunus(DE)**
Erfinder: **Walch, Axel, Dr.**
**Hans-Sachs-Strasse 5**
**D-6000 Frankfurt am Main 90(DE)**
Erfinder: **Wullbrandt, Dieter, Dr.**
**Bienerstrasse 29**
**D-6238 Hofheim am Taunus(DE)**

**Beschreibung**

Die Erfindung betrifft ein Verfahren zur abgasfreien Begasung von Fermentationsmedien mit Sauerstoff oder sauerstoffhaltigen Gasen, wobei der Sauerstoff mittels fluider Trägermedien in das Fermentationsmedium transportiert wird und dort über die Flüssig/Flüssig-Phasengrenze in das Fermentationsmedium eintritt und wobei anschließend das Sauerstoff-ab- und Kohlendioxidangereicherte fluide Trägermedium ohne Emission von Abgasströmen wiederum mit Sauerstoff angereichert wird, wobei das dabei verdrängte Kohlendioxid desorbiert und entfernt wird.

Die Kultivierung von lebenden Zellen, insbesondere in großen Mengen, ist heute ein wesentlicher Bestandteil für die Produktion von beispielsweise antiviralen Substanzen, wie Interferon oder von biologisch aktiven Substanzen, wie Hormonen. Insbesondere bei der Herstellung monoklonaler Antikörper, die die Fähigkeit besitzen, ganz bestimmte Epitope eines Proteins (Makromoleküls) zu erkennen und an diese zu binden, werden sogenannte Hybridoma-Zellen - ein Fusionsprodukt von Antikörper produzierenden Zellen mit Myelom-Zellen - in großen Mengen kultiviert.

In herkömmlichen Laboratoriumsverfahren zur Kultivierung von Zellen werden diese in einer Petrischale in einem Medium suspendiert. Sauerstoff und Kohlendioxid diffundieren durch die Oberfläche des Mediums in die Zellen. Die Tiefe des Kulturmediums beeinflußt die Diffusionsgeschwindigkeit von Sauerstoff und Kohlendioxid zu den Zellen. Mit zunehmender Mediumtiefe nimmt das Verhältnis von Oberfläche zu Volumen des Mediums ab. Zu einem bestimmten Zeitpunkt sind die Sauerstoff- und Kohlendioxidkonzentration in der Luft über der Schale und/oder die Lösegeschwindigkeit in dem Medium ungenügend, um das Gesamtvolumen des Mediums mit genügend Gas zu versorgen und die Erfordernisse der wachsenden Zellen zu befriedigen, d.h. der verfügbare Sauerstoff wird zum begrenzenden Wachstumsfaktor. Dementsprechend lehrt der Stand der Technik, daß die Tiefe des Mediums bei statischen Kulturen im Bereich von mm liegen soll. Im allgemeinen beträgt die obere Grenze der Zelldichte für derartige Kulturen etwa $10^5$ Zellen/ml Kulturmedium, wenn die Luft über dem Kulturmedium als Sauerstoffversorgung verwendet wird.

Werden Verfahren zur Kultivierung von Zellen in großtechnischem Maßstab durchgeführt, so ist es schwierig, ausreichende Sauerstoffmengen, die die Bedürfnisse der Zellen befriedigen, zur Verfügung zu stellen.

Oft werden Gase direkt durch das Medium geperlt, um die Zellen mit den benötigten Gaskonzentrationen zu versorgen. Das direkte Durchperlen ist jedoch vor allem bei der Kultivierung von empfindlichen Zellen ungeeignet. Diese Zellen werden beim Durchperlen des Gases mit einer ausreichenden Geschwindigkeit, um entsprechende Gaskonzentrationen im Medium aufrechtzuerhalten oder durch mechanische Rührer auf Grund der hohen Scherbelastung physikalisch beschädigt. Auch bilden Proteinbestandteile des Mediums, die normalerweise in allen Zellkulturen notwendig sind, auf der Mediumoberfläche einen Schaum, der die Zellen einfangen kann. Die in dem Schaum eingeschlossenen Zellen sterben rasch ab.

Anti-Schaummittel, die dem Medium zugesetzt werden können, sind jedoch gelegentlich für die Zellen der Kultur toxisch und können außerdem bei der Aufarbeitung nur schwer vom Produkt abgetrennt werden.

Eine elegante Methode selbst empfindliche Zellen in großtechnischem Maßstab zu Kultivieren und dabei die nötige Sauerstoffversorgung sicherzustellen, ist der Sauerstoffeintrag in das Kulturmedium mittels eines fluiden Trägermediums. Es ist bekannt, daß beispielsweise Fluorkohlenwasserstoffe Sauerstoff ca. 20 mal besser physikalisch lösen können als Wasser. Werden solche Fluorkohlenwasserstoffe also mit Sauerstoff (bzw. Luft) gesättigt, so lassen sie sich ideal als fluides Trägermedium einsetzen, um Sauerstoff in ein Kulturmedium zu transportieren. Der Sauerstoff wandert dann über die Flüssig/Flüssig-Phasengrenze vom Fluorkohlenwasserstoff in das wäßrige Kulturmedium. Der Sauerstoff-abgereicherte Fluorkohlenwasserstoff kann dan auf Grund seiner nicht Mischbarkeit mit Wasser leicht vom Kulturmedium abgetrennt, mit Sauerstoff wieder angereichert werden und ins Kulturmedium zurückgeführt werden. Solche Verfahren werden beispielsweise beschrieben in der amerikanischen Patentschrift US 3850753. Hier wird aerobe Fermentation bei gleichzeitiger Begasung, Rühren und/oder Schütteln im System Wasser/Wasser nicht mischbarer inerter Lösungsmittel wie perfluor-$C_1$-$C_{20}$-Alkane, beschrieben, S. Wang, Biotechnol. Lett. Vol. 7 (1985) S. 81-86 beschäftigt sich mit der aeroben Fermentation von E. coli BPP01 in Gegenwart von Perfluormethyldecalin. Das organische Lösungsmittel wird von oben nach unten eingedüst und kontinuierlich aus dem System entfernt. Es kommt zu keiner Schaumbildung. In Science, Vol 219, S. 1448-1449 (1983) ist die Verwendung einer polylysinstabilisierten Perfluorkohlenwasserstoffemulsion (FC-70, ein tri-($C_{15}$-Perfluor-Alkyl)-Amin, FC-77, ein Gemisch aus $C_8F_{18}$ und cyclischem $C_8F_{16}O$) beschrieben, die als Microcarrier zur Kultivierung von adhärenten Zellen benutzt wird, bzw. als Blutersatz eingesetzt wird.

Die europäische Patentanmeldung EP 0 164 813 beschreibt eine Methode zur Kultivierung von tierischen oder pflanzlichen Zellen, deren Sauerstoffbedarf während der Fermentation durch eine sauerstoffgesättigte Fluorkohlenwasserstofflösung gedeckt wird. Eine der beiden Phasen - Fermentationsmedium oder fluides Sauerstoff-Trägermedium - kann kontinuierlich betrieben werden.

Bei allen Vorzügen, die die großtechnische Fermentation von lebenden Zellen mit sich bringt, darf ein Aspekt - der vor allem bei der Fermentation von gentechnisch manipulierten Zellen auftritt - nicht außer Acht gelassen werden : Die Abluft, die von solchen Fermentern produziert wird, und die gegebenenfalls auch geringe Mengen Zellmaterial enthält. Gerade bei der Fermentation von gentechnisch modifizierten Mikroorganismen ist mit äußerster Sorgfalt dafür zu sorgen, daß die Abluft kein Zellmaterial mehr enthält. Dies erfordert zur Zeit großen technischen Aufwand (Sicherheitsfermentation).

Aufgabe der Erfindung war es nun, ein Fermentationsverfahren bereitzustellen, bei dem sowohl die Sauerstoffversorgung des Fermentationsmediums in optimaler Weise gewährleistet ist und bei dem gleichzeitig die Auflage der Sicherheitsfermentation (Abgasfreiheit) auf eine wirtschaftliche Art und Weise erfüllt werden.

Diese Aufgabe wird gelöst durch ein

Verfahren zum Eintrag von Sauerstoff in Fermentationsmedien mit Hilfe fluider Trägermedien durch Transport des Sauerstoffs in das Fermentationsmedium über die Flüssig/Flüssig-Phasengrenze, wobei der vom Fermentationsmedium entnommene Sauerstoff im Sauerstoff-abgereicherten Trägermedium in einer Begasungsvorrichtung ohne Emission von Abgasströmen ergänzt wird und das während der Fermentation gebildete Kohlendioxid, welches ebenfalls im Trägermedium gelöst ist, teilweise oder ganz desorbiert und entfernt wird.

Unter Fermentationsmedien werden die allgemeinen üblichen, meist wäßrigen Kulturmedien verstanden, die neben den suspendiert Zellen das Nährmedium mit geeigneten Kohlenstoff- und Stickstoff-Quellen enthalten Kultivierbare Zellen sind beispielsweise tierische, pflanzliche oder mikrobielle Zellen, die auch immobilisiert bzw. enkapsuliert sein können.

Unter fluiden Trägermedien werden solche Flüssigkeiten verstanden, die mindestens

a) mit Wasser wenig oder bevorzugt gar nicht mischbar sind

b) Sauerstoff reversibel besser binden können als Wasser

c) Inert gegenüber den verwendeten Zellen sind

und darüber hinaus bevorzugt noch

d) spezifisch schwerer sind als das Kulturmedium (Wasser)

e) gleichzeitig auch Kohlendioxid reversibel besser binden können als Wasser

Geeignete fluide Trägermedien sind beispielsweise perfluorierte geradkettige oder verzweigte $C_1$-$C_{20}$-Alkane, perfluorierte $C_5$-$C_{14}$-Cycloalkane, perfluoriertes Tetrahydrofuran, perfluoriertes Tetrahydropyran, perfluoriertes Adamantan sowie die mit $C_1$-$C_5$-perfluoralkyl substituierten Perfluor-$C_5$-$C_{14}$-Cycloalkane, Perfluor-Tetrahydrofurane, Perfluor-Tetrahydropyrane und Perfluor-Adamantane aber auch Perfluor-$C_1$-$C_{20}$-Alkoxy-Verbindungen und perfluorierte Polyether wie $C_{10}F_{22}O_2$ (®HOSTINERT 130, $C_{13}F_{28}O_3$ - (HOSTINERT 175), $C_{16}F_{34}O_4$ (HOSTINERT 216) und $C_{22}F_{46}O_6$ (HOSTINERT 272, Hersteller Hoechst AG).

Beispielsweise seien genannt:

Perfluorierte Derivate von Heptan, Octan, Nonan, Tributylamin, N-Methylmorpholin, 1-Methyldecalin, Decalin, Naphthalin, Methyldecalin, Methylnaphthalin, 2-Butyl-, 2-Pentyl-, 2-Hexyl-, 2-Heptylfuran, 2-Butyl-, 2-Pentyl-, 2-Hexyl- oder 2-Heptyltetrahydrofuran.

Geeignet sind aber auch Silicone, wie Dimethyl- oder Phenylmethylsilicone, Polyvinylpyrrolidone oder solche Substanzen, die nicht toxisch gegenüber den Zellen sind und als Blutersatzstoffe verwendet werden können (s. DMW Nr. 35, 105 Jahrgang, Stuttgart, 29.8.1980, Seite 1197-1198 und J. Fluorine Chemistry, 9 (1977), 137-146).

Der Gasaustausch zwischen Fermentationsmedien und Sauerstoffträgermedium erfolgt über die Flüssig/Flüssig-Phasengrenze. Erwiesenermaßen ist hierbei die Sauerstoffübertragungsrate wesentlich höher als bei einem Phasenübergang Gas/Flüssig, wie er etwa beim Durchperlen von Sauerstoff (Luft) durch das Fermentationsmedium erfolgt. Gleichzeitig mit der Abgabe von Sauerstoff an das Fermentationsmedium erfolgt eine Kohlendioxidaufnahme seitens des Trägermediums, da sich meistens auch Kohlendioxid in dem Sauerstoffträgermedium besser (physikalisch) löst, als in der wäßrigen Kulturbrühe.

Bei dem erfindungsgemäßen Verfahren geht man am besten so vor, daß man beispielsweise in einer Blasensäule das fluide Trägermedium mit Sauerstoff oder Luft oder einem Sauerstoff/Luft-Gemisch versetzt. Das dermaßen mit Gas gesättigte fluide Trägermedium wird nun mit dem Kulturmedium in Kontakt gebracht, wobei der oben beschriebene Gasaustausch stattfindet. Dies kann beispielsweise so erfolgen, daß man das spezifisch schwerere Medium von oben nach unten durch eine Flüssigkeitssäule des spezifisch leichteren Mediums tropfen läßt, oder aber das spezifisch leichtere Medium von unten nach oben durch das spezifisch schwerere Medium aufsteigen läßt. In beiden Fällen trennen sich die Phasen wieder, so daß das Sauerstoff-abgereicherte und Kohlendioxidangereicherte fluide Trägermedium abgezogen werden kann und in den Blasenreaktor geleitet werden kann, wo durch den überschüssigen, durchperlenden Sauerstoff (Luft) das physikalisch gelöste Kohlendioxid vertrieben wird und sich das fluide Trägermedium erneut mit Sauerstoff anreichert. Erfindungswesentlich ist, daß das ausgetriebene Kohlendioxid zusammen mit überschüssigem Sauerstoff (Luft) aus dem Gasraum der Blasensäule abgezogen wird und das Kohlendioxid ganz oder teilweise chemisch oder physikalisch desorbiert wird. Dies kann beispielsweise durch einen Alkalimetalllaugen-Wäscher erfolgen, wobei sich festes Alkalimetallcarbonat bildet. Das Restgas (hauptsächlich bestehend aus Sauerstoff) wird erfindungsgemäß wiederum in die Blasensäule geleitet. Somit ist der Gaskreislauf geschlossen und es kann kein Abgas entweichen. Vorteilhaft an diesem Verfahren ist, daß nur der tatsächlich vom Kulturmedium verbrauchte Sauerstoff nachgeführt zu werden braucht. Ein weiterer Vorteil des Verfahrens besteht darin, daß mittels eines Bypasses um den Kohlendioxid-Wäscher die Kohlendioxidkonzentration im Gas und damit im flüssigen Trägermedium und damit wiederum im Kulturmedium kontrolliert werden kann, was eine exakte Steuerung des pH-Wertes ermöglicht.

Vorteilhaft bei dem erfindungsgemäßen Verfahren ist ferner, daß sowohl die Fermentationslösung als auch das fluide Trägermedium kontinuierlich betrieben werden können, d.h. während des Fermentationsprozesses kann sowohl das wässrige Kulturmedium ständig erneuert werden, als auch - gegebenenfalls bei stationärem Fermentationsmedium - das fluide Trägermedium ständig regeneriert werden. Dementsprechend bezeichnet man die jeweilige Phase - das Fermentationsmedium und/oder das fluide Trägermedium - als "kontinuierliche Phase".

Für den Fall, daß das fluide Trägermedium durch eine Flüssigkeitssäule des Fermentationsmediums tropft (unabhängig davon, ob das fluide Trägermedium nun spezifisch leichter oder spezifisch schwerer ist als das Fermentationsmedium), genügt allein der Eintrag des Sauerstoffträgermediums, um eine ausreichende Durchmischung des Fermentationsmediums hervorzurufen. Gegebenenfalls kann aber auch das Fermentationsmedium durch den Einsatz von geeigneten Leitvorrichtungen im Fermenter oder durch einen externen Umlauf (Kreislauf) durchmischt werden.

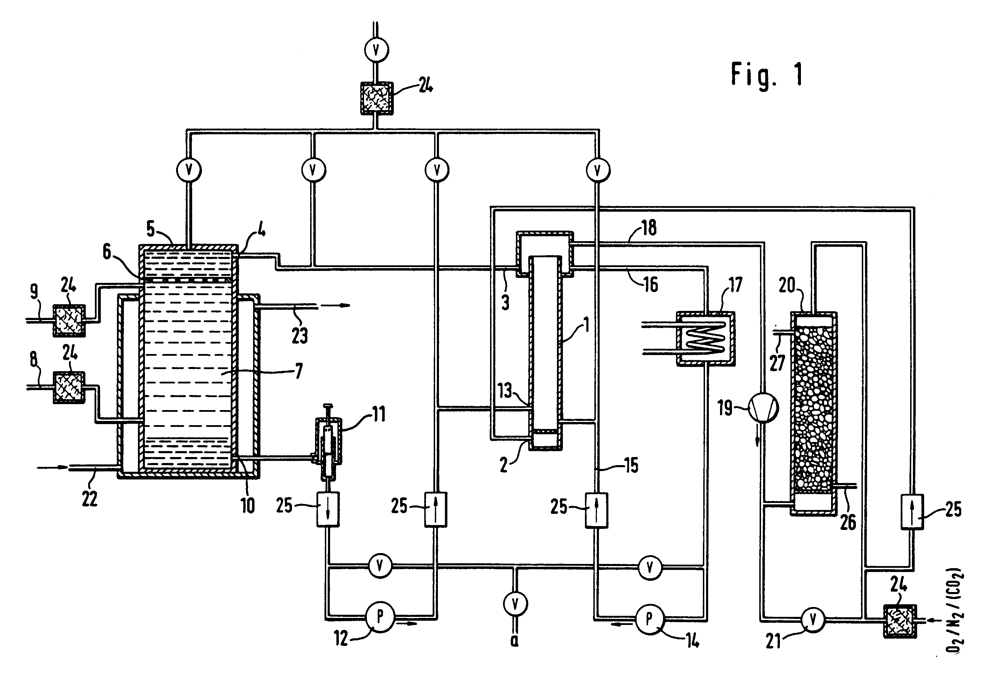

Eine bevorzugte Ausgestaltung des Verfahrens läßt sich mit der in Figur 1 abgebildeten Vorrichtung durchführen. In einer Blasensäule (1) wird ein fluides Sauerstoffträgermedium, welches spezifisch schwerer ist als Wasser, beispielsweise ein Fluorkohlenwasserstoff, über eine Zuführung (2) mit einem hauptsächlich Sauerstoff enthaltenden Gasgemisch versetzt. Über eine Ableitung (3) wird der Sauerstoff-angereicherte Fluorkohlenwasserstoff entnommen und über eine Öffnung (4) in einem temperierbaren Fermenter (5) gegeben. Durch eine Lochplatte (6) ($\phi$ der Löcher zwischen 0,5-4 mm) tropft der Sauerstoffangereicherte Fluorkohlenwasserstoff in das wässrige Kulturmedium (7), welches über die Zu- (8) und Ableitungen (9) gegebenenfalls kontinuierlich gefahren werden kann. Der spezifisch schwerere Fluorkohlenwasserstoff gibt während seines Heruntertropfens Sauerstoff an das wässrige Kulturmedium ab und reichert sich gleichzeitig mit Kohlendioxid aus dem Kulturmedium an. Der so ab-/angereicherte Fluorkohlenwasserstoff wird über eine Öffnung (10) entnommen und über eine Niveauschlaufe (11) (die den Fluorkohlenwasserstoff-Flüssigkeitsspiegel im Fermenter regelt) und eine Pumpe (12) durch die Öffnung (13) wieder in die Blasensäule zurückgeführt. Eine Umwälzpumpe (14) sorgt in Verbindung mit den Zu- und Ableitungen (15) und (16) für eine gute Durchmischung des Fluorkohlenwasserstoffs in der Blasensäule. Über den in den Kreislauf geschalteten Wärmetauscher (17) wird das fluide Trägermedium entsprechend der Fermentationstemperatur eingestellt. Durch den ständig in der Blasensäule herrschenden Sauerstoff-Überdruck wird das in dem zurückgeführten, Sauerstoff-abgereicherten Fluorkohlenwasserstoff vorhandene physikalisch gelöste Kohlendioxid ausgetrieben. Gleichzeitig reichert sich der Fluorkohlenwasserstoff wieder mit Sauerstoff an. Das hauptsächlich Kohlendioxid und überschüssigen Sauerstoff (Luft) enthaltende Abgas wird über eine Leitung (18) entnommen und mittels eines Gebläses (19) einem beispielsweise mit Natronlauge gefüllten Kohlendioxid-Wäscher (20) zugeführt.

Hier wird das Kohlendioxid als Carbonat ausgefällt und das hauptsächlich nur noch Sauerstoff (Luft) enthaltende Gas wird wieder über die Zuleitung (2) der Blasensäule zugeführt. Durch einen Bypaß (21) um den Kohlendioxid-Wäscher kann für eine definierte Kohlendioxid-Konzentration im System gesorgt werden, womit der pH-Wert im Fermenter geregelt werden kann.

Die sterile Anlage wird mit dem fluiden Trägermedium über den Anschluß (a) bei gleichzeitiger Öffnung der Ventile (v) befüllt. Während der Fermentation sind die Ventile (v) geschlossen.
Der Fermenter (5) kann über die Zu- und Ab-Leitungen (22) und (23) temperiert werden. ((24) = Sterilfilter, (25) = Durchflußmesser, (26), (27) = Zu- bzw. Ableitungen zur Befüllung/Entleerung des Kohlendioxidwäschers).

## Beispiele

Die in Figur 1 dargestellte Apparatur wurde für aerobe Fermentationen mit perfluorierten Kohlenwasserstoffen als Sauerstoffträger eingesetzt.

| Fermenter (5) : | Durchmesser 220 mm |
|---|---|
| | Höhe 220 mm |
| Lochscheibe (6) : | Material: V4A-Stahl |
| | Lochdurchmesser 1 mm |
| Gasstrom durch die Blasensäule : | 100 - 500 l/h |

Sauerstoffträger:

Hostinert 216

Molmasse: 902 g/mol; Siedepunkt: 216°C; Oberflächenspannung (25°C): 15,4 mN/m; dynamische Viskosität: 8,78 mPa·s (20°C); $mlO_2$/100 ml: 51,4 (25°C); Dichte: 1,839 kg/l (20°C)

Hostinert 175

Molmasse: 736 g/mol; Siedepunkt: 175°C; Oberflächenspannung (25°C): 14,6 mN/m; dynamische Viskosität: 4,39 mPa·s; (20°C) $mlO_2$/100 ml: 57 (25°C); Dichte: 1,816 kg/l (20°C)
Hostinert-Kreislauf-Strom I: (16)-(14)-(15) [siehe Figur 1] 180 l/h
Hostinert-Kreislauf-Strom II: (4)-(6)-(10) [siehe Figur 1] 20-150 l/h.

## A) Allgemeine Verfahrensangaben

Die sterilisierte Anlage sowie der Reaktor (siehe Figur 1) werden, entsprechend der Niveauschlaufeneinstellung, mit sterilem fluiden Trägermedium befüllt, temperiert und in der Blasensäule mit Stickstoff-Sauerstoff-Mischungen (0 bis 100 % $O_2$, 0 bis 100 % $N_2$) sowie unter Zugabe von Kohlendioxid begast. Der Reaktor wird anschließend mit 10 l Nährlösung gefüllt, temperiert und über den Hostinertstrom (4-6-10) homogen durchmischt sowie mit Sauerstoff angereichert. Der Fermenter wird mit der Vorkultur angeimpft und während der Fermentation Parameter wie pH-Wert, Substrat- und Produktkonzentration kontrolliert und gesteuert. Der gelöst-Sauerstoffgehalt wird über den Hostinertstrom (4-6-10) gesteuert. Das während der Fermentation gebildete Kohlendioxid kann in dem Wäscher (20) ganz oder teilweise entfernt und der verbrauchte Sauerstoff nachdosiert werden.

## Beispiel 1

Fermentation von Escherischia coli K 12

| Nährmedium: | 0,8 % Fleischpepton (tryptisch verdaut) |
|---|---|
| | 0,8 % Caseinpepton (tryptisch verdaut) |
| | 0,3 % Hefeextrakt |
| | 0,5 % Natriumchlorid |
| | 1 % Glucose |
| Temperatur: | 37°C; pH-Wert: 7,0 |

Die Fermentation wurde entsprechend der unter A beschriebenen Weise durchgeführt. Als fluides Trägermedium diente Hostinert 216, das mit Luft in der Blasensäule gesättigt wird.

Das Fermentationsmedium wird mit 100 ml Vorkultur angeimpft und die Sauerstoffversorgung über den Eintrag von Hostinert 216 (60 l/h) sichergestellt. Entsprechend dem Zellwachstum in der exponentiellen Wachstumphase wird der Hostinert-Strom bis zu 150 l pro Stunde erhöht, wobei die Sättigung der dispersen Phase mit einer Mischung aus Stickstoff-Sauerstoff vorgenommen wird.

In der exponentiellen Wachstumsphase wird eine Generationszeit von 30 Minuten bei einem Trockenmassegehalt von 0,32 g/l am Ende der exponentiellen Phase erreicht.

## Beispiel 2

Fermentation von Backhefe (Saccharomyces cerevisiae)

Synthetisches Medium mit 1 % Glucose und
2,48 g/l $(NH_4)_2HPO_4$
0,4 g/l $MgSO_4$ · 7 $H_2O$

0,2 g/l KCl

0,12 g/l $CaCl_2$ • 2 $H_2O$

20 mg/l NaCl

20 mg/l m-Inosit

1,2 mg/l Ca-Pantothenat

0,8 mg/l $H_3BO_3$

0,4 mg/l $ZnSO_4$ • 7 $H_2O$

0,4 mg/l $MnSO_4$ • 7 $H_2O$

0,2 mg/l $FeCl_3$ • 6 $H_2O$

0,2 mg/l $Na_2MoO_4$ • 2 $H_2O$

0,12 mg/l NaJ

0,04 mg/l $CuSO_4$ • 5 $H_2O$

0,02 mg/l Biotin.

Die Fermentationslösung und das ®Hostinert 216 werden auf 30°C temperiert und der pH-Wert während der Fermentation durch Zugabe von 1 normaler Natronlauge bei 5,0 konstant gehalten. Nach dem Animpfen mit 100 ml Vorkultur (24 Stunden alt, synthetisches Medium mit 1 % Glucose) wird der Hostinertfluß durch den Reaktor von 6 l/h auf 120 l/h eingestellt und die Fermentation mit einer Verdünnungsrate von $D = 1\ h^{-1}$ (Zuflußrate f = 1 l/h, Anschlüße (8), (9)) kontinuierlich betrieben. Nach der Gleichgewichtseinstellung beträgt die Biomasse 4,5 g/l.

**Beispiel 3**

Entsprechend dem Beispiel 11 der deutschen Patentanmeldung P 37 060 10.4, auf die an dieser Stelle ausdrücklich Bezug genommen wird, werden Hybridoma-Zellen mikroenkapsuliert.

Der Reaktor wird mit Dulbecco's-Medium gefüllt, auf 37°C temperiert und die Fermentationslösung mit ®Hostinert 175, das mit einer Mischung aus Stickstoff-Sauerstoff (1:1) mit 5 % Kohlendioxid gesättigt ist, mit Sauerstoff angereichert. Das Fermentationsmedium wird mit 10 ml Kapseln (Kapseldurchmesser 500 $\mu$m; $10^4$ Kapseln) pro Liter angeimpft und während der Fermentation das Medium diskontinuierlich gewechselt.

| Tag | Zellzahl pro ml Reaktorvolumen | $\mu$g Antikörper pro ml Reaktorvolumen |
|---|---|---|
| 3 | $2 \cdot 10^5$ | 8 |
| 4* | $4 \cdot 10^5$ | 20 |
| 5 | $9,6 \cdot 10^5$ | 30 |
| 8* | $7 \cdot 10^5$ | 50 |
| 10 | $1,7 \cdot 10^6$ | 110 |
| 13 | $1,04 \cdot 10^6$ | 90 |

* an diesen Tagen wurde das Medium gewechselt.

Der Antikörpergehalt (Maus IgG) in Gew.-% wurde mittels Enzymimmunoassay (Behring Werke AG, Marburg) bestimmt.

**Patentansprüche**

1. Verfahren zum Eintrag von Sauerstoff in ein Fermentationsmedium in einem geschlossenen System mit Hilfe fluider Trägermedien und Kontrolle der Kohlendioxidkonzentration im Gasstrom, fluidem Trägermedium und Fermentationsmedium, dadurch gekennzeichnet, daß

a) ein fluides Trägermedium mit dem Fermentationsmedium in Kontakt gebracht wird, wobei Sauerstoff aus dem fluiden Trägermedium in das Fermentationsmedium übergeht und gleichzeitig Kohlendioxid vom Fermentationsmedium in das fluide Trägermedium übergeht,

b) das von Sauerstoff abgereicherte und mit Kohlendioxid angereicherte fluide Trägermedium wird zu einer Blasensäule transportiert in der das fluide Trägermedium mit Sauerstoff angereichert wird und Kohlendioxid aus dem fluiden Trägermedium ausgetrieben wird, das ausgetriebene Kohlendioxid und überschüssiger Sauerstoff aus der Blasensäule bilden einen Abgasstrom,

c) das mit Sauerstoff angereicherte fluide Trägermedium wird zum Fermentationsmedium zurück transportiert,

d) der Abgasstrom aus der Blasensäule wird zu einem Kohlendioxid-Wäscher transportiert, wo das Kohlendioxid ganz oder teilweise aus dem Abgasstrom entfernt wird und somit einen Restabgasstrom bildet,

e) die Kohlendioxidkonzentration in dem Restabgasstrom wird geregelt, und

f) der Restabgasstrom wird zur Blasensäule zurück transportiert, wobei kein Gas in die Umgebung emittiert wird.

2. Verfahren nach Anspruch 1, dadurch gekennzeichnet, daß der Abgasstrom im Kreis gefahren wird, wobei dem Abgasstrom das Kohlendioxid ganz oder teilweise entzogen wird.

3. Verfahren nach Anspruch 1 oder 2, dadurch gekennzeichnet, daß das fluide Trägermedium eine höhere Dichte als das Fermentationsmedium besitzt.

4. Verfahren nach Anspruch 1 oder 2, dadurch gekennzeichnet, daß das fluide Trägermedium eine geringere Dichte als das Fermentationsmedium besitzt.

5. Verfahren nach einem oder mehreren der Ansprüche 1 bis 4, dadurch gekennzeichnet, daß entweder das fluide Trägermedium durch das Fermentationsmedium oder das Fermentationsmedium durch das fluide Trägermedium in Form kleiner Tropfen perlt.

6. Verfahren nach Anspruch 5, dadurch gekennzeichnet, daß die Tropfendispergierung des fluiden Trägermediums oder des Fermentationsmediums durch Kapillaren und/oder durch ein Loch- und/oder Siebboden erfolgt.

7. Verfahren nach Anspruch 6, dadurch gekennzeichnet, daß die Kapillaren, die Löcher oder Maschen des Siebs einen Durchmesser von 0,5-4 mm aufweisen.

8. Verfahren nach einem oder mehreren der Ansprüche 1 bis 7, dadurch gekennzeichnet, daß als fluides Trägermedium perfluorierte Kohlenwasserstoffe, perfluorierte Polyether, Perfluoralkoxyverbindungen, Silicone, Polyvinylpyrrolidon oder Blutersatzstoffe verwendet werden.

9. Verfahren nach einem oder mehren der Ansprüche 1 bis 8, dadurch gekennzeichnet, daß die Vermischung des Fermentationsmediums durch den Einsatz von Leitvorrichtungen und/oder durch externen Umlauf gesteuert wird.

10. Verfahren nach einem oder mehreren der Ansprüche 1 bis 9, dadurch gekennzeichnet, daß die Fermentation kontinuierlich betrieben wird.

11. Verfahren nach einem oder mehreren der Ansprüche 1 bis 10, dadurch gekennzeichnet, daß Mikroorganismen, Säugerzellen, Pflanzenzellen, gentechnologisch modifizierte Spezies oder immobilisierte oder enkapsulierte Zellen der vorbeschriebenen Art, kultiviert werden.

## Claims

1. A method for introducing oxygen into a fermentation medium in a closed system with the aid of fluid carrier media and monitoring of the carbon dioxide concentration in the gas stream, fluid carrier medium and fermentation medium wherein,

a) a fluid carrier medium is brought into contact with the fermentation medium, whereupon oxygen is transferred from the fluid carrier medium into the fermentation medium and simultaneously carbon dioxide is transferred from the fermentation medium into the fluid carrier medium,

b) the oxygen-depleted and carbon dioxide-enriched fluid carrier medium is transported to a bubble column in which the fluid carrier medium is enriched with oxygen, and carbon dioxide is driven out of the fluid carrier medium, the carbon dioxide which has been driven out and excess oxygen from the bubble column form an exit gas stream,

c) the oxygen-enriched fluid carrier medium is transported back to the fermentation medium,

d) the exit gas stream from the bubble column is transported to a carbon dioxide scrubber where the carbon dioxide is completely or partially removed from the exit gas stream and thus forms a residual exit gas stream,

e) the carbon dioxide concentration in the residual exit gas stream is controlled, and

f) the residual exit gas stream is transported back to the bubble column with no gas being emitted to the environment.

2. The method as claimed in claim 1, wherein the exit gas stream is recycled with the carbon dioxide being entirely or partially removed from the exit gas stream.

3. The method as claimed in claim 1 or 2, wherein the fluid carrier medium has a higher density than the fermentation medium.

4. The method as claimed in claim 1 or 2, wherein the fluid carrier medium has a lower density than the fermentation medium.

5. The method as claimed in one or more of claims 1 to 4, wherein either the fluid carrier medium passes in the form of small drops through the fermentation medium, or the fermentation medium passes in the form of small drops through the fluid carrier medium.

6. The method as claimed in claim 5, wherein the dispersion into drops of the fluid carrier medium or of the fermentation medium is carried out by capillaries and/or by a perforated and/or sieve plate.

7. The method as claimed in claim 6, wherein the capillaries, the perforations or the sieve mesh have a diameter of 0.5 - 4 mm.

8. The method as claimed in one or more of claims 1 to 7, wherein perfluorinated hydrocarbons, perfluorinated polyethers, perfluoroalkoxy compounds, silicones, polyvinylpyrrolidone or blood substitutes are used as fluid carrier medium.

9. The method as claimed in one or more of claims 1 to 8, wherein the mixing of the fermentation medium is controlled by the use of conducting devices and/or by an external circulation.

10. The method as claimed in one or more of claims 1 to 9, wherein the fermentation is operated continuously.

11. The method as claimed in one or more of claims 1 to 10, wherein microorganisms, mammalian cells, plant cells, species which have been modified by genetic engineering, or immobilized or encapsulated cells of the type described above, are cultivated.

**Revendications**

1. Procédé pour l'introduction d'oxygène dans un milieu de fermentation dans un sytème clos, à l'aide de milieux vecteurs fluides et de réglage de la concentration du dioxyde de carbone dans le courant gazeux, le milieu vecteur fluide et le milieu de fermentation, caractérisé en ce que

a) on met un milieu vecteur fluide en contact avec le milieu de fermentation, grâce à quoi de l'oxygène passe du milieu vecteur fluide dans le milieu de fermentation et en même temps du dioxyde de carbone passe du milieu de fermentation dans le milieu vecteur fluide,

b) le milieu vecteur fluide appauvri en oxygène et enrichi en dioxyde de carbone est envoyé dans une colonne de barbotage, dans laquelle le milieu vecteur fluide est enrichi en oxygène et le dioxyde de carbone est chassé du milieu vecteur fluide, le dioxyde de carbone chassé et l'oxygène en excès provenant de la colonne de barbotage forment un courant de gaz rejeté,

c) le milieu vecteur fluide enrichi en oxygène est renvoyé au milieu de fermentation,

d) le courant de gaz rejeté provenant de la colonne de barbotage est envoyé à un laveur de dioxyde de carbone, dans lequel le dioxyde de carbone est en partie ou en totalité éliminé du courant de gaz rejeté, et il se forme ainsi un courant de gaz rejeté résiduel,

e) on ajuste la concentration du dioxyde de carbone dans le courant de gaz rejeté résiduel, et

EP 0 317 854 B1

f) le courant de gaz rejeté résiduel est renvoyé à la colonne de barbotage, grâce à quoi aucun gaz n'est émis dans l'environnement.

2. Procédé selon la revendication 1, caractérisé en ce que le courant de gaz rejeté est mis en circuit, grâce à quoi le dioxyde de carbone est en partie ou en totalité extrait du courant de gaz rejeté.

3. Procédé selon la revendication 1 ou 2, caractérisé en ce que le milieu vecteur fluide a une densité supérieure à celle du milieu de fermentation.

4. Procédé selon la revendication 1 ou 2, caractérisé en ce que le milieu vecteur fluide a une densité inférieure à celle du milieu de fermentation.

5. Procédé selon une ou plusieurs des revendications 1 à 4, caractérisé en ce que soit le milieu vecteur fluide traverse sous forme de petites gouttes le milieu de fermentation, soit le milieu de fermentation traverse sous forme de petites gouttes le milieu vecteur fluide.

6. Procédé selon la revendication 5, caractérisé en ce que la dispersion en gouttes du milieu vecteur fluide ou du milieu de fermentation s'effectue par des capillaires et/ou à travers un plateau perforé et/ou à tamis.

7. Procédé selon la revendication 6, caractérisé en ce que les capillaires, les trous ou les ouvertures de mailles du tamis présentent un diamètre de 0,5-4 mm.

8. Procédé selon une ou plusieurs des revendications 1 à 7, caractérisé en ce que, en tant que milieu vecteur fluide, on utilise des hydrocarbures perfluorés, des polyéthers perfluorés, des composés perfluoro-alcoxy, des silicones, la polyvinylpyrrolidone ou des substituts du sang.

9. Procédé selon une ou plusieurs des revendications 1 à 8, caractérisé en ce que le mélangeage du milieu de fermentation est régi par l'utilisation de dispositifs de conduite et/ou par circulation externe.

10. Procédé selon une ou plusieurs des revendications 1 à 9, caractérisé en ce que la fermentation est effectuée en continu.

11. Procédé selon une ou plusieurs des revendications 1 à 10, caractérisé en ce que l'on cultive des microorganismes, des cellules mammaliennes, des cellules végétales, des espèces modifiées par génie génétique ou des cellules immobilisées ou encapsulées du type précédemment décrit.

9

Fig. 1